(19) **Europäisches Patentamt / European Patent Office / Office européen des brevets**

(11) **EP 3 769 320 B1**

(12) **EUROPEAN PATENT SPECIFICATION**

(45) Date of publication and mention
of the grant of the patent:
**06.05.2026 Bulletin 2026/19**

(21) Application number: **19714130.2**

(22) Date of filing: **19.03.2019**

(51) International Patent Classification (IPC):
**G16H 40/67** *(2018.01)*     **G16H 40/63** *(2018.01)*
**G16H 10/60** *(2018.01)*     **G16H 50/30** *(2018.01)*
**G16H 50/70** *(2018.01)*

(52) Cooperative Patent Classification (CPC):
**G16H 50/30; G16H 10/60; G16H 40/63;**
**G16H 40/67; G16H 50/70**

(86) International application number:
**PCT/EP2019/056744**

(87) International publication number:
**WO 2019/179963 (26.09.2019 Gazette 2019/39)**

(54) **A SYSTEM AND METHOD FOR DETERMINING ALLERGENS WHICH MAY PRESENT A RISK OF A RESPIRATORY ATTACK TO A USER OF THE SYSTEM**

SYSTEM UND VERFAHREN ZUR BESTIMMUNG VON ALLERGENEN, DIE EIN RISIKO EINES RESPIRATORISCHEN ANGRIFFS AUF EINEN BENUTZER DES SYSTEMS DARSTELLEN KÖNNEN

SYSTÈME ET PROCÉDÉ DE DÉTERMINATION D'ALLERGÈNES, QUI PEUVENT PRÉSENTER UN RISQUE D'ATTAQUE RESPIRATOIRE À UN UTILISATEUR DU SYSTÈME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2018 PCT/CN2018/080025**
**23.08.2018 EP 18190370**

(43) Date of publication of application:
**27.01.2021 Bulletin 2021/04**

(73) Proprietor: **Versuni Holding B.V.**
**5656 AE Eindhoven (NL)**

(72) Inventors:
• **SU, Jing**
**5656 AE Eindhoven (NL)**
• **KONG, Tao**
**5656 AE Eindhoven (NL)**

(74) Representative: **Vollering, Stefanus Franciscus Maria**
**Philips Domestic Appliances IP Department**
**High Tech Campus 42**
**5656 AE Eindhoven (NL)**

(56) References cited:
**WO-A1-2017/148876**     **US-A1- 2014 213 925**
**US-A1- 2016 256 097**

**Description**

FIELD OF THE INVENTION

This invention relates to a system and method for determining allergens that may present a risk to a user. Such allergens may include plant pollen, animal dander, dust and mold.

BACKGROUND OF THE INVENTION

The spread of respiratory allergies (allergic rhinitis) is increasing. Around one third of adults in the US has respiratory allergies and up to 40% of children in the US are allergic to respiratory allergens. Pollen, dust mites, mold and pet dander are allergen sources for respiratory allergies.

There are many different types of pollen as well as many different examples of the other types of allergen which can cause allergies. Without visiting a clinician, allergy suffers are unlikely to know which types of airborne allergen they are allergic to. People who have allergic rhinitis generally visit a clinician for allergen screening. This normally involves a skin prick test or immunoglobulin E (IgE) blood test. This process is time-consuming and not pleasant, especially for children.

Even if a subject does know which types of specific allergen they react to, it is still a problem to avoid allergic reactions effectively, for a number of reasons.

First, there are many different types of airborne allergens. Only pollen concentration is widely reported and forecast. However, a pollen forecast (in the newspapers, on a smartphone app or on TV) is very generic and often does not give sufficient information to particular individuals to know if the pollen presents a danger to them. For example, a high pollen count does not necessarily mean that any particular individual will be affected, because there are many different types of pollen, from different kinds of trees, from grass and from a variety of weeds. A high overall pollen count does not necessarily indicate a strong concentration of a specific pollen to which an individual will react. Conversely, a low pollen count does not mean there is a low concentration of a particular allergen of concern to a particular individual. For airborne allergens other than pollen, no objective data is available.

Second, it is not clear when and where an individual is likely to be exposed to a particular allergen. There are many opportunities for subjects to inhale airborne allergens, like inhaling outdoor pollen, contacting dust mites carried by colleagues or classmates, inhaling indoor mold, etc. However, people seldom know when and where they are at risk to inhaling allergens, as the visible allergic reaction typically occurs at some point in the 24 hour period after inhaling the allergen.

US2014213925A1 discloses devices, systems, kits and methods for measuring peak expiratory or inspiratory flow-rate and dynamically predicting respiratory episodes. Additionally, systems for analyzing and processing the measurement in a communication networked environment are also provided. An aspect of the disclosure is directed to a respiratory device, **In** some configurations the respiratory device comprises a housing adaptable and configurable to communicate with an electronic device, a mouth piece having a proximal end and a distal end configurable to engage a mouth of a patient and transmit an air flow, one or more diaphragm sensors configured to detect a breath vibration from the air flow in the mouth piece, and a processor adaptable and configurable to analyze the breath vibration detected by the one or more diaphragm sensors.

US20160256097A1 discloses information from a sensor that is local to a user is received. The information may indicate that the user has experienced a physiological event. The information is analyzed to determine whether the physiological event should be classified as an allergic reaction. **If** the physiological event should be classified as an allergic reaction, an action is taken such as particles currently present in the environment local to the user are collected.

There is therefore a need for a system and method which is better able to determine when and where people are likely to be exposed to allergens which affect them, and to be able to identify those allergens.

SUMMARY OF THE INVENTION

The invention is defined by the claims.

According to examples in accordance with an aspect of the invention, there is provided a system for determining allergens which may present a risk of a respiratory attack to a user of the system, comprising:

a processor;
a first input to the processor for receiving an indication that a respiratory attack has taken place;
a location indicator; and
a second input to the processor for receiving historical data for a time period which precedes the respiratory attack, wherein the historical data relates to locations of the user during the time period, including at least environmental factors which comprise plant types known to be present in the vicinity of the locations,
wherein the processor is adapted to process the historical data thereby to:

determine the location at which a respiratory attack is most likely to have been triggered by assigning weights to the environmental factors; and
identify a set of most likely plant types by applying weightings to each plant type relating at least to the amount of the plant type present at the determined location; and
output said set of plant types as those which may present a risk of a respiratory attack to the user of the system.

This system is able to identify possible allergens for the user of the system without performing a skin prick test or requiring an expensive IgE blood test. It uses statistical analysis of the conditions leading up to previous respiratory attacks, in particular the combination of location information and environmental factors at those locations. Some of this information may be input to the system partly manually by a user and other information may be provided automatically, for example the location information (from a GPS system or other indication of location), pollen count information (from an external database) and plant information (from an external database e.g. of crop locations). The various factors are weighted so that a most likely location which caused a subsequent respiratory attack may be determined.

From this location information, a most likely set of plants is determined which may be the cause of the respiratory attack at that location.

A most basic implementation of the system is able to identify plant types for which pollen is an allergen. The system may however also identify animal breeds causing an allergy, or identify that mold or dust present a risk to a particular user.

Based on the information collected and determined by the system, it is first possible to identify the allergens likely to be a risk factor for the user, but it is also then possible to indicate where and when the specific user of the system is likely to become exposed to their specific allergens. It is thus possible to indicate how to avoid becoming in contact with those specific allergens.

The environmental factors relate to weather conditions at the locations visited by the individual. The environmental factors may further comprise weather conditions at the locations and pollen count information at the locations, and optionally also indoor conditions comprising indoor humidity and/or room ventilation rate and/or indoor temperature. Thus, the indoor and outdoor environments in the locations visited by the individual are monitored.

The weather conditions of the environmental factors comprise one or more of:

> outdoor humidity;
> wind speed;
> wind direction;
> temperature.

This weather condition information enables a risk of allergen exposure at a certain location to be assessed.

The historical data may further comprise contact factors which comprise animals known to be present at the locations and optionally also people known to be present at the locations. Weights are then also assigned to the contact factors for the purposes of determining the location. Different people may be assigned different weighting, for example according to the pets that they have or other information about those individuals, such as the plants that they have in their garden. This information is typically provided manually by the user to the system. The system may then also identify animal types which are the cause of allergic reactions. Thus, animals and plants may both be treated in the same way by the system.

The processor is for example adapted to identify the set of most likely plant types by applying weightings to each plant type relating at least to the amount of the plant type present at the determined location. Thus, once a location has been identified, if a plant is the possible cause (rather than a pet, for example) the most likely plant types are determined based on information about that particular location.

In this way, there is a two stage process for identifying the allergen source. First the most likely location is derived from historical information about all locations visited by the user of the system, and then for the particular location a statistical analysis of possible plant types is carried out.

The weightings for each plant type for example relate to the environmental factors (pollen count, pollution levels, wind information) and a general known allergenicity level for that plant type.

For one respiratory attack, a set of most likely causes is thus identified. The processor is preferably adapted to identify a more accurate set of most likely plant types by combining the sets of most likely plant types from multiple preceding respiratory attacks. In this way, the likely causes of an attack can be narrowed with the benefit of additional information.

The time period over which monitoring is appropriate for example has a maximum duration of 48 hours, and monitoring may be performed for a period of time of between 6 and 36 hours leading up to an attack or a time period between 12 and 36 hours. A short duration will give low data storage requirement and a long duration will give better accuracy but requires more data storage. 24 hours is the typical maximum time period which may elapse before a respiratory attack is evident after an exposure to an allergen.

In addition to identifying likely allergens for the user, the processor may also be adapted to provide recommendations for locations for the user to avoid a respiratory attack.

Examples in accordance with another aspect of the invention provide a method for determining allergens which may present a risk level of a respiratory attack to a user of the system, comprising:

> tracking the location of the user;
> receiving an input indicating a respiratory attack;
> receiving historical data for a time period which precedes a respiratory attack, wherein the historical data relates to locations of the user during the time period, including at least environmental factors which comprise plant types known to be present at the locations; and

processing the historical data thereby to:

determine the location at which a respiratory attack is most likely to have been triggered by assigning weights to the environmental factors; and

identify a set of most likely plant types by applying weightings to each plant type relating at least to the amount of the plant type present at the determined location; and

output said set of plant types as those which may present a risk of a respiratory attack to the user of the system.

The environmental factors may further comprise weather conditions at the locations and pollen count information at the locations, and optionally also indoor conditions comprising indoor humidity and/or room ventilation rate and/or indoor temperature and the weather conditions of the environmental factors comprise one or more of outdoor humidity, wind speed, wind direction and temperature.

The method may comprise identifying a set of most likely plant types by applying weightings to each plant type relating at least to the amount of the plant type present at the determined location.

The method may comprise identifying a set of most likely plant types by combining the sets of most likely plant types from multiple preceding respiratory attacks.

The method may also be used to provide recommendations for locations and actions for the user to avoid a respiratory attack.

The historical data may further comprise contact factors which comprise animals known to be present at the locations and optionally also people known to be present at the locations.

The invention may be implemented at least in part by software, and thus provides a computer program comprising computer program code means which is adapted, when said program is run on a computer, to implement the method defined above.

BRIEF DESCRIPTION OF THE DRAWINGS

Examples of the invention will now be described in detail with reference to the accompanying drawings, in which:

Figure 1 shows a system for identifying allergens of risk to a user;

Figure 2 shows a method for identifying allergens of risk to a user; and

Figure 3 illustrates an example of a computer for implementing the processor of the system of Figure 1.

DETAILED DESCRIPTION OF THE EMBODIMENTS

The invention provides a system and method for determining allergens which may present a risk of a respiratory attack to a user of the system. Historical data which precedes a respiratory attack is statistically analyzed. The data relates to the locations where the user has been present and includes environmental factors and optionally also human or animal contact factors. A location at which a respiratory attack is most likely to have been triggered is derived as well as a set of most likely plant types (or animal types, mold or dust mites) to have caused the respiratory attack.

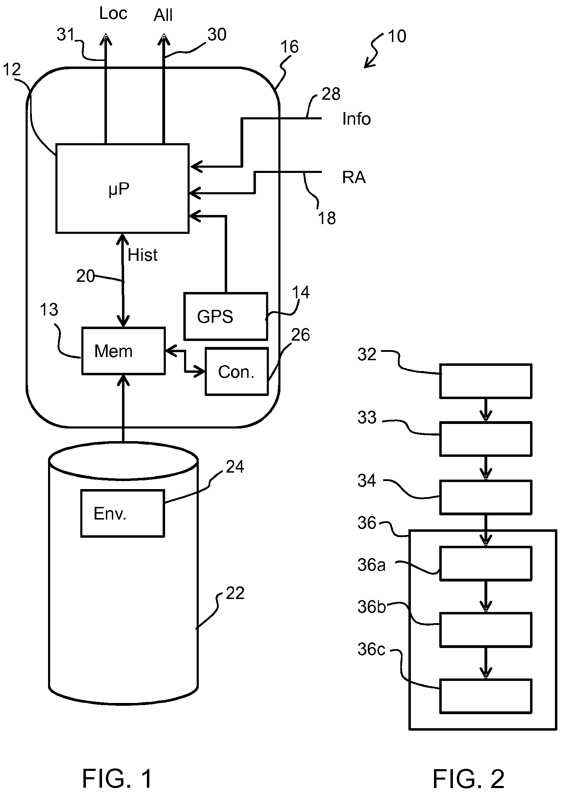

Figure 1 shows a system 10 in accordance with one example of the invention. The system shown is associated with (and carried by) a particular user.

The system 10 is for determining allergens which may present a risk of a respiratory attack to a user of the system. It comprises a processor 12 and a location indicator 14 for identifying or tracking the location of the user. The location indicator is for example a location sensor such as a GPS sensor. However, other methods of identifying location may be used, such as imaging information, for example images from Google Glass. These images may also enable location to be tracked over the time period of interest.

A memory 13 is used for storing data over a moving time window, so that historical data is available when needed.

The processor and location indicator may be part of a mobile telephone or tablet 16. Thus, the invention may be implemented by loading suitable software onto a mobile telephone, which may already include all the hardware necessary to implement the system of the invention.

The processor has a first input 18 for receiving an indication that a respiratory attack ("RA") has taken place and a second input 20 for receiving historical data ("Hist") for a time period which precedes the respiratory attack. This data is received from the memory 13. When a user has a respiratory attack such as an asthma attack, a simple emergency button may be pressed by the user. This enables the system to take a capture of the historical data.

The processor 12 determines, from the historical data, the location at which a respiratory attack is most likely to have been triggered. For the determined location, a most likely allergen or set of possible allergens is then derived.

The historical data 20 is received from the memory, which in turn obtains the data from a database 22, for example by connection over the internet. The historical data 20 is basically information which affects the probability of being exposed to allergens in dependence on location.

The historical data in particular includes environmental factors 24 and optionally also contact factors 26.

The environmental factors are for example part of the data in the database and comprise plant types known to be present in the vicinity of the locations. Thus, the database stores a map of plant crop varieties and locations. The environmental factors also include weather conditions at the locations and pollen count information at the locations. Particle concentration levels may also be monitored.

The weather conditions comprise one or more of outdoor humidity, wind speed, wind direction and temperature. These may all be obtained from the database 22, but some or all may equally be obtained by local sensors forming part of the system, such as a temperature sensor, a relative humidity sensor and a particulate concentration sensor. These are all outdoor weather conditions, but the environmental factors may further comprise indoor conditions such as indoor humidity and/or room ventilation rate and indoor particulate concentration. This information may be obtained from local sensors, or for example by communication with a building management system.

Thus, the indoor and outdoor environments in the locations visited by the individual are monitored over time. These environmental factors enable a risk of allergen exposure at a certain location to be assessed.

The historical data also preferably includes contact factors 26, for example relating to pet interaction which takes place at the location. However, it may include interaction with people, for example when the pets of those other people are identified as risk factors associated with those people.

This contact information is more specific to the individual and is therefore less able to be obtained from a shared database 22. Instead, the processor has a third input 28 for receiving information ("Info") from the user which provides a higher degree of tailoring of the system to the particular user. For this reason, the contact factors 26 are shown as part of the device 16 rather than the database 22.

The processor first determines the most likely location where the exposure to an allergen took place based on the historical information.

To do this, the environmental factors and optionally also the contact factors are each assigned a weighting, and the time during which the location was visited is also taken into account. There is a weighting allocated to each factor which is taken into account.

Each factor is converted into a weighting, for example from 1 to 5 (other scales may be used, such as 1, 3, 5, 7 so the highest weighting has more influence).

For example, a wind speed below 5km/h may have a weighting of 1 and a wind speed above 50km/h may have a weighting of 5, with the intermediate wind speeds divided into the remaining bands 2 to 4. A particle concentration (such as PM2.5 or PM10) may be treated in the same way. For example, a PM2.5 concentration below $5\mu g/m^3$ may correspond to a weighting of 1 and a PM2.5 concentration above $50\mu g/m^3$ may correspond to a weighting of 5.

All other factors which are measured as a numerical scale may be treated in the same way, such as temperature, humidity, room ventilation rate, pollen count.

Humidity is for example a key indicator for allergen release. High humidity could cause allergen release from pollen and enable pollen attachment to other small particles. The allergen on small particles has higher chance to be inhaled.

Note that some factors may not have the full range of possible values 1 to 5, and furthermore there may only be some values of interest. For example, temperature may be considered to contribute less to the risk of an allergen attach (it may for example relate to mold spores), so only a narrow temperature range (e.g. 22 - 35°C) may be relevant, and even then only a weighting of 3 may be appropriate.

Other factors may be more binary in nature, and thus have a weighting of only 1 or 5 (or other value) such as pet interaction. Different animals may receive a different weighting. Furthermore, presence in the location where the pet lives will receive a higher weighting than contact with a person who has a pet in their home (i.e. only indirect contact with their pet).

Plants present in the vicinity of the location may have a weighting according to the distance of the crop to the nearest location occupied. Thus, the weighting for plants being nearby may be a function of the distance to the known location of the plants. Different plants may also have a different weighting according to their known general allergenicity.

The weightings may all be multiplied together (which is why in the example above the lowest score is 1) to provide an overall score.

This overall score applies to a particular location when those particular conditions are present. Examples of the locations are at home indoors, at home outdoors, on the commute to work and at work indoors. Different contact factors are present at the different locations such as the immediate family at home, pets at home, and the colleagues at work (giving indirect contact to their associated pets).

The time at which the user is present at each location is also taken into account. and it is used as a multiplier. Thus, for a particular location, the overall score is multiplied by the time for which that overall score is applicable. Over the duration of the time the user is at the location, the overall score may evolve. The final score for that location is then the combination of the individual time periods. **In** other words, a total integrated score over time is obtained.

The time period may be as simple as an actual time duration (scaled so that the multiplication by time is appropriate, i.e. a nominal time period has value of 1). However, the time period may also be defined with weighted values, for example particularly if the weightings are not calculated every second or minute. For example, a time period in the range 0-30 minutes may be a weighting of 1; 30 minutes to 1 hour may be a weighting of 3 and more than 1 hour may have a weighting of 5. The weighting applied will ensure that the effect of the time duration is correctly taken into account compared to the other weighting values.

The allergen risk level A for that location (i.e. the final score) becomes:

$$A = \sum_i \ W_i \ x \ t_i$$

$W_i$ is the overall score (i.e. risk level) during a time period $t_i$ and the product is summed to provide a final score over the full time period when present at the location.

From the allergen risk levels for the different locations visited by the user before a respiratory attack, a most likely location which caused the attack is in this way identified.

Note that the approach above is only one example. Basically, the approach involves taking multiple risk factors into account which can be assessed for a particular location, and deriving a risk level for that location. Risk levels for different locations can then be compared. The way factors contribute to risk levels can be determined based on analysis of data for multiple system users or from experimental work. A default setting for the weightings may be used, which may then evolve as more data is collected.

For the identified location, a set of most likely plant (or indeed animal) types to have caused the respiratory attack is determined based on the historical data associated with the determined location. Note that there may be more than one location identified. For example rather than seeking the location with the highest risk score, multiple locations with a risk score exceeding a threshold may be analyzed in the following steps of the method.

For example, a single location most likely to be the cause may be identified as the commute to work (where "location" is used broadly, and may include a journey or scenario, i.e. a set of time-sequential locations).

The processor then identifies a set of most likely plant types responsible for the allergens on the commute to work (there may be no animals associated with this journey).

This involves using the plant information along the commute route together with the the related allergic factors for each kind of plant including its amount, pollen count, pollen allergenicity level, local environmental factors such as wind and air pollution level, humidity and distance to the plants.

This basically involves dividing the overall risk factor A for the commute into the factors for the different plants that are experienced at that location (i.e. journey).

By assigning weights to these factors, in the manner explained above, a probability value can be assigned to the different plant types that have been in the vicinity of the user.

A most likely set of possible allergen sources is then determined according to the probability, for example a set of 5 most likely allergen sources.

Each plant type may have different weightings for the amount of plant present, the wind speed, the allergenicity level, the pollen count and the particulate air pollution level, because these factors may correlate differently with allergen risk for different plants. For example, wind speed will affect different plant pollens differently.

Each plant type may then have its own allergen risk score which is the product of these weighting factors. Again, with a lowest weighting of 1, the corresponding factor will have no influence on the risk score.

Thus, the first step provides a general risk level for a location (or journey) whereas the second step provides a specific risk level associated with each plant.

The system eventually outputs a list of probable allergens (shown as the output 30, "All"). It may also indicate locations to be avoided, shown as output 31 ("Loc") or indeed it may generate routes (for example as part of a navigation system) which avoid locations where the identified allergens are known to be present.

To continuously maintain accuracy, the model (i.e. the database content and the weighting scores) can be improved by data learning. Furthermore, the algorithm operated by the processor may be updated (e.g. by input 28) by an exclusion function so that it excludes allergens and allergen sources that are already known not to be triggers for the user. This will provide a personalized allergen estimation model.

First, the data learning is used to customize weighting of each factor as explained above for the particular user. Second, the exclusion function is used to narrow down the possible allergens in long term monitoring.

The information for the exclusion function may be input by the user as mentioned above, but equally it may be determined based on analysis of the date for a set of allergic reactions.

For one respiratory attack, a set of most likely causes is identified. The processor may identify a set of most likely plant (or animal) types or locations which given an exposure to dust mites by combining the sets of most likely plant (or animal) types and other information from multiple preceding respiratory attacks. For example, if there are possible allergens A to G, and the top five are identified at each respiratory attack, there should be overlap of the actual causes. If one allergen type only appears infrequently in the list, it can be discarded and identified as a type which the user does not react to. This analysis may of course be based on a more rigorous statistical analysis. By excluding allergens from the analysis, calculation amount and time is reduced.

This system is in this way able to identify possible allergens for the user of the system without performing a skin prick test or requiring an expensive IgE blood test. It uses statistical analysis of the conditions leading up to previous respiratory attacks, in particular the combination of location information and environmental and optional contact factors at those locations. It is also possible for the system to indicate where and when the specific user of the system is likely to become exposed to their specific allergens. It is thus possible to indicate how to avoid becoming in contact with those specific allergens.

As mentioned above, a core aspect of the system is to enable a general pollen allergy to be refined to identify specific pollen allergies. In addition, the system may identify allergy to mold, dust, mites or other allergens.

By way of example, key indicators for dust and mold can be obtained from the PM10 concentration and indoor humidity. Thus, indoor humidity and PM10 concentration may form part of the historical data so that these risk factors may also be identified.

The weighting rule for relative humidity is that a higher humidity corresponds to a larger weighting value and a higher PM10 concentration corresponds to a larger weighting value.

An indication of cleaning frequency may also be used. Frequent cleaning corresponds to a lower weighting. Indoor cleaning information may be obtained in automated way from wireless communication with a floor care device, or it may be obtained based on detected fluctuations in PM10 data.

If the indoor humidity in the location is always higher than 80% and cleaning frequency is low, there is an increased likelihood of indoor mold.

All of these various factors may be monitored. As a minimum, the system has the capability to identify different pollen types. However, in an extended implementation, it may identify an allergy of any of the various different types described above.

The time period for example has a duration of between 6 hours and 36 hours for example a full 24 hour period. This is the typical time period which may elapse before a respiratory attack is evident after an exposure to an allergen.

Figure 2 shows a method for determining allergens which may present a risk level of a respiratory attack to a user of the system, comprising:

in step 32, tracking the location of the user;
in step 33, receiving an input indicating a respiratory attack (RA);
in step 34, receiving historical data for a time period which precedes the respiratory attack, wherein the historical data relates to locations of the user during the time period.

As explained above, the historical data includes environmental factors which comprise plant types known to be present at the locations, weather conditions at the locations and pollen count information at the locations; and optionally also contact factors which comprise animals known to be present at the locations.

In step 36, the historical data is processed.

In step 36a the location at which a respiratory attack is most likely to have been triggered is determined by assigning weights to the environmental factors and contact factors if used.

In step 36b, a set of most likely plant types to have caused the respiratory attack is determined based on the historical data associated with the determined location.

In step 36c, recommendations are provided for locations for the user to avoid a respiratory attack.

The method may identify the main risk allergens as plants or animals or a combination of both. They are processed in the same way, with corresponding weightings.

The system may be divided into different parts in different ways. For example there are various sensors used, including a location indicator (such as a GSM signal processor for triangulation, or a GPS or other satellite positioning system or an imaging device which can indicate the location based on visualization), and optional other sensors such as temperature, humidity and pollution sensors. There may be other sensors such as physiological sensors. These sensors may all be part of the system, or else some sensors may be part of other devices (with other primary functions) with which the system communicates.

The system described above makes use of a processor 12 for processing data.

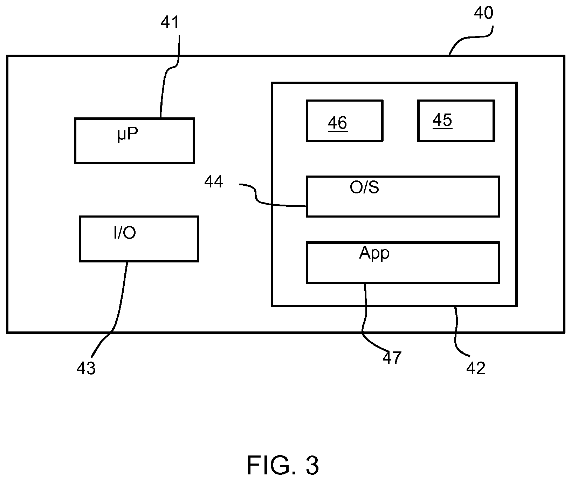

Figure 3 illustrates an example of a computer 40 for implementing the processor described above.

The computer 40 includes, but is not limited to, PCs, workstations, laptops, PDAs, palm devices, servers, storages, and the like. Generally, in terms of hardware architecture, the computer 40 may include one or more processors 41, memory 42, and one or more I/O devices 43 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 41 is a hardware device for executing software that can be stored in the memory 42. The processor 41 can be virtually any custom made or commercially available processor, a central processing unit (CPU), a digital signal processor (DSP), or an auxiliary processor among several processors associated with the computer 40, and the processor 41 may be a semiconductor based microprocessor (in the form of a microchip) or a microprocessor.

The memory 42 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 42 may incorporate electronic, magnetic, optical, and/or other types of storage media. Note that the memory 42 can have a distributed architecture, where various components are situated remote from one another, but can be accessed by the processor 41.

The software in the memory 42 may include one or more separate programs, each of which comprises an ordered listing of executable instructions for implementing logical functions. The software in the memory 42 includes a suitable operating system (O/S) 44, compiler 45, source code 46, and one or more applications 47 in accordance with exemplary embodiments.

The application 47 comprises numerous functional components such as computational units, logic, functional units, processes, operations, virtual entities, and/or modules.

The operating system 44 controls the execution of computer programs, and provides scheduling, input-output control, file and data management, memory management, and communication control and related services.

Application 47 may be a source program, executable program (object code), script, or any other entity comprising a set of instructions to be performed. When a source program, then the program is usually translated via a compiler (such as the compiler 45), assembler, interpreter, or the like, which may or may not be included within the memory 42, so as to operate properly in connection with the operating system 44. Furthermore, the application 47 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 43 may include input devices such as, for example but not limited to, a mouse, keyboard, scanner, microphone, camera, etc. Furthermore, the I/O devices 43 may also include output devices, for example but not limited to a printer, display, etc. Finally, the I/O devices 43 may further include devices that communicate both inputs and outputs, for instance but not limited to, a network interface controller (NIC) or modulator/demodulator (for accessing remote devices, other files, devices, systems, or a network), a radio frequency (RF) or other transceiver, a telephonic interface, a bridge, a router, etc. The I/O devices 43 also include components for communicating over various networks, such as the Internet or intranet.

When the computer 40 is in operation, the processor 41 is configured to execute software stored within the memory 42, to communicate data to and from the memory 42, and to generally control operations of the computer 40 pursuant to the software. The application 47 and the operating system 44 are read, in whole or in part, by the processor 41, perhaps buffered within the processor 41, and then executed.

When the application 47 is implemented in software it should be noted that the application 47 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The invention may be used as part of a pollution mask or air purifier system or HVAC system or dehumidifier.

Other variations to the disclosed embodiments can be understood and effected by those skilled in the art in practicing the claimed invention, from a study of the drawings, the disclosure, and the appended claims. **In** the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

**Claims**

1. A system for determining allergens which may present a risk of a respiratory attack to a user of the system, comprising:

    a processor (12);
    a first input (18) to the processor for receiving an indication that a respiratory attack has taken place;
    a location indicator (14); and
    a second input (20) to the processor for receiving historical data for a time period which precedes the respiratory attack, wherein the historical data relates to locations of the user during the time period, including at least environmental factors (24) which comprise at least plant types known to be present in the vicinity of the locations,

wherein the processor (12) is adapted to process the historical data thereby to:

> determine the location at which a respiratory attack is most likely to have been triggered by assigning weights to the environmental factors;
> identify a set (30) of most likely plant types by applying weightings to each plant type relating at least to the amount of the plant type present at the determined location; and output said set (30) of plant types as those which may present a risk of a respiratory attack to the user of the system.

2. A system as claimed in claim 1, wherein the historical data further comprises contact factors (26) which comprise animals known to be present at the locations and optionally also people known to be present at the locations, and wherein weights are also assigned to the contact factors.

3. A system as claimed in claim 1 or 2, wherein the environmental factors (24) further comprise weather conditions at the locations and pollen count information at the locations, and optionally also indoor conditions comprising indoor humidity and/or room ventilation rate and/or temperature.

4. A system as claimed in claim 3, wherein the weather conditions of the environmental factors comprise one or more of:

> outdoor humidity;
> wind speed;
> wind direction;
> temperature.

5. A system as claimed in claim 4, wherein the weightings for each plant type further relate to the environmental factors and a pollen allergenicity level.

6. A system as claimed in any preceding claim, wherein the processor (12) is adapted to identify a set of most likely plant types by combining the sets of most likely plant types from multiple preceding respiratory attacks.

7. A system as claimed in any preceding claim, wherein the time period has a duration of between 6 hours and 36 hours.

8. A system as claimed in any preceding claim, wherein the processor (12) is adapted to provide recommendations (31) for locations for the user to avoid a respiratory attack.

9. A method for determining allergens which may present a risk level of a respiratory attack to a user of the system, comprising:

> (32) tracking the location of the user;
> (33) receiving an input indicating a respiratory attack;
> (34) receiving historical data for a time period which precedes a respiratory attack, wherein the historical data relates to locations of the user during the time period, including at least environmental factors which comprise plant types known to be present at the locations;
> (36) processing the historical data thereby to:
>
>> (36a) determine the location at which a respiratory attack is most likely to have been triggered by assigning weights to the environmental factors; and
>> (36b) identify a set of most likely plant types by applying weightings to each plant type relating at least to the amount of the plant type present at the determined location; and
>
> output said set of plant types as those which may present a risk of a respiratory attack to the user of the system.

10. A method as claimed in claim 9, wherein the environmental factors further comprise weather conditions at the locations and pollen count information at the locations, and optionally also indoor conditions comprising indoor humidity and/or room ventilation rate and/or indoor temperature and the weather conditions of the environmental factors comprise one or more of outdoor humidity, wind speed, wind direction and temperature.

11. A method as claimed in any one of claims 9 to 10, comprising identifying a set of most likely plant types by combining the sets of most likely plant types from multiple preceding respiratory attacks.

12. A method as claimed in any one of claims 9 to 11, wherein the historical data further comprises contact factors which comprise animals known to be present at the locations and optionally also people known to be present at the locations.

13. A computer program comprising computer program code which is adapted, when said program is run on a computer, to implement the method of any one of claims 9 to 12.

**Patentansprüche**

1. System zur Bestimmung von Allergenen, die für einen Benutzer des Systems ein Risiko für einen Atemnotanfall darstellen können, umfassend:

einen Prozessor (12);

einen ersten Eingang (18) zu dem Prozessor zum Empfangen einer Anzeige, dass ein Atemnotanfall stattgefunden hat;

einen Aufenthaltsortindikator (14); und

einen zweiten Eingang (20) zu dem Prozessor zum Empfangen historischer Daten für einen Zeitraum vor dem Atemnotanfall, wobei sich die historischen Daten auf Aufenthaltsorte des Benutzers während des Zeitraums beziehen, einschließlich mindestens Umweltfaktoren (24), die mindestens Pflanzenarten umfassen, von denen bekannt ist, dass sie in der Nähe der Aufenthaltsorte vorhanden sind,

wobei der Prozessor (12) so ausgelegt ist, dass er die historischen Daten verarbeitet, um dadurch:

den Aufenthaltsort zu bestimmen, an dem ein Atemnotanfall am wahrscheinlichsten ausgelöst wurde, indem den Umweltfaktoren Gewichte zugewiesen werden;

einen Satz (30) wahrscheinlichster Pflanzenarten zu identifizieren, indem jeder Pflanzenart eine Gewichtung zugewiesen wird, die sich mindestens auf die Menge der Pflanzenart an dem bestimmten Aufenthaltsort bezieht; und

den Satz (30) von Pflanzenarten als jene auszugeben, die für den Benutzer des Systems ein Risiko für einen Atemnotanfall darstellen könnten.

2. System nach Anspruch 1, wobei die historischen Daten ferner Kontaktfaktoren (26) umfassen, die Tiere umfassen, von denen bekannt ist, dass sie sich an den Aufenthaltsorten aufhalten, und optional auch Personen, von denen bekannt ist, dass sie sich an den Aufenthaltsorten aufhalten, und wobei den Kontaktfaktoren auch Gewichte zugeordnet werden.

3. System nach Anspruch 1 oder 2, wobei die Umweltfaktoren (24) ferner Wetterbedingungen an den Aufenthaltsorten und Informationen zur Pollenkonzentration an den Aufenthaltsorten sowie optional auch Innenraumbedingungen umfassen, die Innenraumfeuchtigkeit und/oder Raumlüftungsrate und/oder Temperatur umfassen.

4. System nach Anspruch 3, wobei die Wetterbedingungen der Umweltfaktoren eines oder mehrere umfassen von:

Luftfeuchtigkeit im Freien;
Windgeschwindigkeit;
Windrichtung;

Temperatur.

5. System nach Anspruch 4, wobei die Gewichtungen für jede Pflanzenart ferner mit den Umweltfaktoren und dem Pollenallergenitätsgrad in Zusammenhang stehen.

6. System nach einem vorstehenden Anspruch, wobei der Prozessor (12) so ausgelegt ist, dass er einen Satz von wahrscheinlichsten Pflanzenarten durch Kombinieren der Sätze wahrscheinlichster Pflanzenarten aus mehreren vorstehenden Atemnotanfällen identifiziert.

7. System nach einem vorstehenden Anspruch, wobei die Zeitspanne zwischen 6 und 36 Stunden liegt.

8. System nach einem vorstehenden Anspruch, wobei der Prozessor (12) so ausgelegt ist, dass er Empfehlungen (31) für Aufenthaltsorte bereitstellt, an denen der Benutzer einen Atemnotanfall vermeiden kann.

9. Verfahren zur Bestimmung von Allergenen, die für einen Benutzer des Systems ein Risiko für einen Atemnotanfall darstellen können, umfassend:

(32) Verfolgen des Aufenthaltsorts des Benutzers;

(33) Empfangen eines Eingangs, der auf einen Atemnotanfall hinweist;

(34) Empfangen von historischen Daten für einen Zeitraum vor einem Atemnotanfall, wobei sich die historischen Daten auf Aufenthaltsorte des Benutzers während dieses Zeitraums beziehen, einschließlich zumindest Umweltfaktoren, die Pflanzenarten umfassen, von denen bekannt ist, dass sie an den Aufenthaltsorten vorkommen;

(36) Verarbeiten der historischen Daten, um dadurch:

(36a) den Aufenthaltsort zu bestimmen, an dem ein Atemnotanfall am wahrscheinlichsten ausgelöst wurde, indem den Umweltfaktoren Gewichte zugewiesen werden; und

(36b) einen Satz wahrscheinlichster Pflanzenarten zu identifizieren, indem jeder Pflanzenart eine Gewichtung zugewiesen wird, die sich mindestens auf die Menge der Pflanzenart an dem bestimmten Aufenthaltsort bezieht; und

den Satz von Pflanzenarten als jene auszugeben, die für den Benutzer des Systems ein Risiko für einen Atemnotanfall darstellen könnten.

**10.** Verfahren nach Anspruch 9, wobei die Umweltfaktoren ferner Wetterbedingungen an den Aufenthaltsorten und Informationen zur Pollenkonzentration an den Aufenthaltsorten sowie optional auch Innenraumbedingungen umfassen, die Innenraumfeuchtigkeit und/oder Raumlüftungsrate und/oder Innentemperatur umfassen, und die Wetterbedingungen der Umweltfaktoren eines oder mehrere von Luftfeuchtigkeit im Freien, Windgeschwindigkeit, Windrichtung und Temperatur umfassen.

**11.** Verfahren nach einem der Ansprüche 9 bis 10, umfassend Identifizieren eines Satzes wahrscheinlichster Pflanzenarten durch Kombinieren der Sätze wahrscheinlichster Pflanzenarten aus mehreren vorangegangenen Atemnotanfällen.

**12.** Verfahren nach einem der Ansprüche 9 bis 10, wobei die historischen Daten ferner Kontaktfaktoren umfassen, die Tiere, von denen bekannt ist, dass sie sich an den Aufenthaltsorten aufhalten, und optional auch Personen, von denen bekannt ist, dass sie sich an den Aufenthaltsorten aufhalten, umfassen.

**13.** Computerprogramm, das Computerprogrammcode umfasst, der dazu ausgelegt ist, wenn das Programm auf einem Computer ausgeführt wird, das Verfahren nach einem der Ansprüche 9 bis 12 zu implementieren.

**Revendications**

**1.** Système de détermination des allergènes qui peuvent présenter un risque de crise respiratoire pour un utilisateur du système, comprenant :

un processeur (12) ;
une première entrée (18) au processeur pour recevoir une indication qu'une crise respiratoire s'est produite ;
un indicateur de lieu (14) ; et
une seconde entrée (20) au processeur pour recevoir des données historiques pour une période de temps qui précède la crise respiratoire, dans lequel les données historiques se rapportent à des lieux de l'utilisateur pendant la période de temps, incluant au moins des facteurs environnementaux (24) qui comprennent au moins des types de plantes connus pour être présents à proximité des lieux,
dans lequel le processeur (12) est adapté pour traiter les données historiques afin de :

déterminer le lieu auquel une crise respiratoire est la plus susceptible d'avoir été déclenchée en attribuant des poids aux facteurs environnementaux ;

identifier un ensemble (30) de types de plantes les plus probables en appliquant des pondérations à chaque type de plante se rapportant au moins à la quantité du type de plante présente sur le lieu déterminé ; et
délivrer en sortie ledit ensemble (30) de types de plantes comme étant ceux qui peuvent présenter un risque de crise respiratoire pour l'utilisateur du système.

**2.** Système selon la revendication 1, dans lequel les données historiques comprennent en outre des facteurs de contact (26) qui comprennent des animaux connus pour être présents sur les lieux et, facultativement, également des personnes connues pour être présentes sur les lieux, et dans lequel des poids sont également attribués aux facteurs de contact.

**3.** Système selon la revendication 1 ou 2, dans lequel les facteurs environnementaux (24) comprennent en outre des conditions météorologiques sur les lieux et des informations de densité pollinique sur les lieux et, facultativement, également des conditions intérieures, comprenant l'humidité intérieure et/ou le taux de ventilation de la pièce et/ou la température.

**4.** Système selon la revendication 3, dans lequel les conditions météorologiques des facteurs environnementaux comprennent une ou plusieurs :

de l'humidité extérieure ;
de la vitesse du vent ;
de la direction du vent ;
de la température.

**5.** Système selon la revendication 4, dans lequel les pondérations pour chaque type de plante se rapportent en outre aux facteurs environnementaux et à un niveau d'allergénicité du pollen.

**6.** Système selon une quelconque revendication précédente, dans lequel le processeur (12) est adapté pour identifier un ensemble de types de plantes les plus probables en combinant les ensembles de types de plantes les plus probables de multiples précédentes crises respiratoires.

**7.** Système selon une quelconque revendication précédente, dans lequel la période de temps présente une durée comprise entre 6 heures et 36 heures.

**8.** Système selon une quelconque revendication précédente, dans lequel le processeur (12) est adapté pour fournir des recommandations (31) concernant les lieux auxquels l'utilisateur peut éviter une crise respiratoire.

**9.** Procédé de détermination des allergènes qui peuvent présenter un niveau de risque de crise respiratoire pour un utilisateur du système, comprenant :

(32) le suivi du lieu de l'utilisateur ;

(33) la réception d'une entrée indiquant une crise respiratoire ;

(34) la réception de données historiques pour une période de temps qui précède une crise respiratoire, dans lequel les données historiques se rapportent à des lieux de l'utilisateur pendant la période de temps, incluant au moins des facteurs environnementaux qui comprennent des types de plantes connus pour être présents sur les lieux ;

(36) le traitement des données historiques afin de

(36a) déterminer le lieu auquel une crise respiratoire est la plus susceptible d'avoir été déclenchée en attribuant des poids aux facteurs environnementaux ; et

(36b) identifier un ensemble de types de plantes les plus probables en appliquant des pondérations à chaque type de plante se rapportant au moins à la quantité du type de plante présente sur le lieu déterminé ; et délivrer en sortie ledit ensemble de types de plantes comme étant ceux qui peuvent présenter un risque de crise respiratoire pour l'utilisateur du système.

10. Procédé selon la revendication 9, dans lequel les facteurs environnementaux comprennent en outre des conditions météorologiques sur les lieux et des informations de densité pollinique sur les lieux et, facultativement, également des conditions intérieures, comprenant l'humidité intérieure et/ou le taux de ventilation de la pièce et/ou la température intérieure et les conditions météorologiques des facteurs environnementaux comprennent une ou plusieurs de l'humidité extérieure, de la vitesse du vent, de la direction du vent et de la température.

11. Procédé selon l'une quelconque des revendications 9 à 10, comprenant l'identification d'un ensemble de types de plantes les plus probables en combinant les ensembles de types de plantes les plus probables de multiples précédentes crises respiratoires.

12. Procédé selon l'une quelconque des revendications 9 à 10, dans lequel les données historiques comprennent en outre des facteurs de contact qui comprennent des animaux connus pour être présents sur les lieux et, facultativement, aussi des personnes connues pour être présentes sur les lieux.

13. Programme informatique comprenant un code de programme informatique qui est adapté, lorsqu'il est exécuté sur un ordinateur, pour mettre en œuvre le procédé selon l'une quelconque des revendications 9 à 12.

FIG. 1

FIG. 2

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014213925 A1
- US 20160256097 A1